# EUROPEAN PATENT SPECIFICATION

(11) **EP 2 659 004 B1**
(45) Date of publication and mention of the grant of the patent: **30.08.2017**
(21) Application number: 11853773.7
(22) Date of filing: 29.11.2011
(51) Int. Cl.: C12Q 1/68

(54) **MODULATION OF DYNEIN IN SKIN**
MODULATION VON DYNEIN IN DER HAUT
MODULATION DE LA DYNÉINE DANS LA PEAU

(30) Priority: 30.12.2010 US 201061428272 P
(43) Date of publication of application: 06.11.2013
(60) Divisional application: 15152603.5
(73) Proprietor: Avon Products, Inc., New York, NY 10017 (US)
(72) Inventor: ZHENG, Qian, Morris Plains, New Jersey 07950 (US); LYGA, John W., Basking Ridge, New Jersey 07920 (US); WYBORSKI, Russell J., Pine Island, New York 10969 (US)
(74) Representative: Dr. Weitzel & Partner
(86) International application number: PCT/US2011/062279
(87) International publication number: WO 2012/091832

(56) References cited:
- WO-A2-2004/026249
- WO-A2-2009/134404
- US-A1- 2003 032 771
- US-A1- 2005 136 028
- US-A1- 2007 010 657
- US-A1- 2009 301 508
- H Randolph Byers ET AL: "Role of Cytoplasmic Dynein in Perinuclear Aggregation of Phagocytosed Melanosomes and Supranuclear Melanin Cap Formation in Human Keratinocytes", Journal of Investigative Dermatology, 1 October 2003 (2003-10-01), pages 813-820, XP55108831, United States DOI: 10.1046/j.1523-1747.2003.12481.x Retrieved from the Internet: URL:http://search.proquest.com/docview/210 347093
- BYERS ET AL.: 'Role of Cytoplasmic Dynein in Perinuclear Aggregation of Phagocytosed Melanosomes and Supranuclear Melanin Cap Formation in Human Keratinocytes.' J. INVEST. DERMATOL. vol. 121, 2003, pages 813 - 820, XP055108831
- BYERS ET AL.: 'Role of Cytoplasmic Dynein in Melanosome Transport in Human Melanocytes.' J. INVEST. DERMATOL. vol. 114, 2000, pages 990 - 997, XP002292324

## Description

### FIELD OF INVENTION

The present invention is defined by the appended claims. In particular, the invention relates to compounds that modulate levels of dynein in skin.

### BACKGROUND

Dyneins are a family of proteins that move along microtubules in the direction of the minus-end. Dyneins convert chemical energy from ATP hydrolysis into mechanical energy needed for step-wise movement along microtubules. Axonemal dynein, found only in cells having cilia and flagella, causes the sliding of microtubules in the axonemes of those structures. Cytoplasmic dynein, on the other hand, is believed to be present in all animal cells. It was identified and characterized much more recently than axonemal dynein. See Paschal B.M., Shpetner H.S., Vallee R.B., "MAP 1C is a microtubule-activated ATPase which translocates microtubules in vitro and has dynein-like properties," J. Cell Biol., 1987 Sep; 105(3):1273-82; and Shpetner H.S., Paschal B.M., and Vallee R.B., "Characterization of the microtubule-activated ATPase of brain cytoplasmic dynein (MAP 1C)," J. Cell Biol., 1988 Sep; 107(3):1001-9.WO2004026249 A1, US2005/136028 A1 and WO2009134404 A2, describe methods and compounds for improving skin appearance. Byers et al., J. Invest. Dermatology, vol. 121, pp.813-820, discusses the influence of dynein for UV protection and melanosome transport.

Cytoplasmic dyneins are cytoskeletal molecular motors that are responsible for mitosis, cell polarization, intracellular transport of vesicles, endosomes, and lysosomes, and other movement within the cell. Cytoplasmic dynein has a molecular mass of about 1.5 Megadaltons (MDa) and has twelve or more polypetide subunits comprising two identical heavy chains of about 530 kDa per chain, two intermediate chains of about 74 kDa, four intermediate chains between about 53 and 59 kDa, and several light chains of about 10 to about 14 kDa. The heavy chains each contain four ATP-binding sites, including the ATP-hydrolytic site, as well as a microtubule binding site. The intermediate chains are believed to bind the dynein to its cargo.

Because the dynein proteins generate the movement of a wide variety of materials in cells, they are essential for many cellular and developmental functions, including, organelle movement, localization of developmental determinants, and potentially long-range signaling in neurons. Dynein is also shown to regulate centrosome reorientation, cytoskeleton remodeling, leading edge formation, and cell migration during wound healing of monolayer cell culture. They also mediate the perinuclear aggregation of phagocytosed melanosomes in human keratinocytes, which lead to the formation of supranuclear melanin.

Deficiencies in the functioning of dynein have been implicated in neuronal degeneration in motor neuron diseases caused by impaired axonal transport in these neurons. Disruption to the genes that encode the cytoplasmic dynein chains has been linked to respiratory disease, primary ciliary diskenesia (Kartagener Syndrome), and may be implicated in motor neuron diseases such as amyotrophic lateral sclerosis. To date, the role of dynein in maintaining healthy skin and slowing skin aging has not been recognized

It is an object of the disclosure to provide compositions and methods for treating, ameliorating, inhibiting and/or preventing dermatological signs of aging. It is the object of the invention to provide methods for treating, ameliorating, inhibiting and/or preventing dermatological signs of aging by modulating the levels of dynein in skin cells. It is a further object of the invention to provide methods for identifying compounds that are useful for treating, ameliorating, inhibiting and/or preventing dermatological signs of aging, based on the ability to modulate the levels of dynein in skin cells.

The foregoing discussion is presented solely to provide a better understanding of nature of the problems confronting the art and should not be construed in any way as an admission as to prior art.

### SUMMARY OF THE INVENTION

In accordance with the foregoing objectives and others, the present invention provides modulators of dynein, preferably cytoplasmic dynein, to improve one or more signs of dermatological aging. The modulators may either upregulate or down regulate levels of dynein in the skin, depending on the desired effect. It is believed that up-regulators of dynein produced within dermal fibroblasts and keratinocytes will lead to increased protein production within the cells, including the fibroblast specific proteins collagen, elastin, and fibrillin, and the keratinocyte protein keratin. Given the importance of fibroblast-produced proteins to overall skin strength and health, upregulation of dyneins will have a beneficial effect on reducing the appearance of aging on skin.

The inventions also embraces modulators which down-regulate levels of dyneins in the skin. Because dynein also mediates the perinuclear aggregation of phagocytosed melanosomes in human keratinocytes, which lead to the formation of supranuclear melanin, down-regulators of dynein, particularly in keratinocytes and/or melanocytes, will be useful for treating age spots and other forms of hyper-pigmentation in the skin. It is also contemplated that down-regulators of dynein will be effective in the treatment of proliferative disorders or the skin, including psoriasis.

In one aspect of the invention, a method is provided for screening candidate substances to identify actives useful for improving the aesthetic appearance of skin. The method comprises assaying candidate substances for ability to modulate dynein expression in a dermal fibroblast or keratinocyte. The method typically involves incubating human dermal fibroblasts or keratinocyte with a candidate substance and subsequently measuring the levels of mRNA encoding cytoplasmic dynein heavy chain subunit DYNC1H1 by a quantitative technique such as quantitative polymerase chain reaction (qPCR)

Methods are therefore provided for improving the aesthetic appearance of human skin comprising topically applying to an area of the skin in need thereof an effective amount of a substance that modulates dynein levels in human dermal fibroblast and keritoncytes. The dynein modulators are referred to herein as "actives" and may be either up-regulators or down-regulators, and typically will be formulated in a cosmetically acceptable vehicle and topically applied to a human integument, such as the skin of the face, neck, hands, chest, legs, etc., for a time sufficient to enhance the health or aesthetic appearance thereof.

In one aspect of the invention, a method is provided for improving the aesthetic appearance of human skin comprising topically applying to an area of the skin in need thereof an effective amount of an active agent that modulates (e.g., up-regulates or down-regulates) cellular levels of cytoplasmic dynein, wherein the ability of the active agent to modulate cytoplasmic dynein has been determined by an assay which measures the expression level of cytoplasmic dynein in a cell that has been contacted with the active agent. The cell used in the assay may be any cell that expresses dynein, but is preferably mammalian, and is more preferably a human or mouse cell. The cell may be a skin cell, such as a fibroblast or keratinocyte. The assay may measure the levels of any fragment or marker of dynein, including the intact complex, but typically what is measured are the expression levels of the cytoplasmic dynein heavy chain subunit DYNC1H1, which is expressed by the human gene *DYNC1H1* or the mouse gene *Dync1h1.* The expression levels of the human gene *DYNC1H1* or the mouse gene *Dync1h1* may be determined, for example, by measuring the expression levels of the corresponding mRNA by any suitable technique, such as quantitative polymerase chain reaction (qPCR).

A method of treating wrinkles and/or fines lines is provided, comprising topically applying to an area of the skin in need thereof an effective amount of an active agent that up-regulates dynein, wherein the ability of the active agent to modulate cytoplasmic dynein has been determined by an assay which measures the expression level of cytoplasmic dynein in a cell that has been contacted with the active agent. The assaying step most typically comprises incubating human dermal fibroblasts with a candidate substance and subsequently measuring the levels of mRNA encoding cytoplasmic dynein heavy chain subunit DYNC1H1, wherein the candidate substance was selected for use as an active agent, in part, on the basis of its ability to up-regulate dynein expression.

A method of treating hyperpigmentation is also provided, comprising topically applying to an area of the skin in need thereof an effective amount of an active agent that down-regulates dynein, wherein the ability of the active agent to modulate cytoplasmic dynein has been determined by an assay which measures the expression level of cytoplasmic dynein in a cell that has been contacted with the active agent. The assay step in this implementation, usually comprises incubating human dermal keratinocytes with a candidate substance and subsequently measuring the levels of mRNA encoding cytoplasmic dynein heavy chain subunit DYNC1H1, wherein the candidate substance was selected for use as an active agent, in part, on the basis of its ability to down-regulate dynein expression.

### DETAILED DESCRIPTION OF THE INVENTION

All terms used herein are intended to have their ordinary meaning unless otherwise provided. By "cosmetically acceptable" is meant that a particular component is generally regarding as safe and non-toxic at the levels employed. The term "prevent," as used herein, includes delaying the onset or progression of a particular sign of skin aging. The term "thin skin" includes skin that becomes thinner with chronological aging as well as prematurely thinned skin, which may be caused, for example, by photo-aging. The phrase "individual in need thereof" refers to a human that could benefit from improved dermal appearance or health, including males or females. The term "skin" includes, without limitation, the lips, skin of the face, hands, arms, neck, and chest. As used herein, the term "consisting essential of" is intended to limit the invention to the specified materials or steps and those that do not materially affect the basic and novel characteristics of the claimed invention, as understood from a reading of this specification.

As used herein, the term dynein refers to cytoplasmic dynein as well as axonemal dynein, although the preferred modulators according to the invention will modulate levels of cytoplasmic dynein. Cytoplasmic dynein includes cytoplasmic dynein 1 complex, as well as cytoplasmic dynein 2 complex, although the preferred modulators according to the invention will modulate levels of cytoplasmic dynein 1 complex. The dynein may be from any animal, but is typically human or mouse dynein, and preferably human dynein. The nomenclature used herein to described cytoplasmic dynein is that of Pfister et al., "Cytoplasmic dynein nomenclature," J. Cell Biol., 2005 November 7; 171(3): 411-413 which is summarized in Table 1.

**Table 1.**

| Gene Symbol | | Protein symbol |
|---|---|---|
| human | mouse | human and mouse |
| (cytoplasmic dynein 1 complex) | | |
| *DYNC1H1* | *Dync1h1* | DYNC1H1 |
| *DYNC1I1* | *Dync1i1* | DYNC1I1 |
| *DYNC1I2* | *Dync1i2* | DYNC1I2 |
| *DYNC1LI1* | *Dync1li1* | DYNC1LI1 |
| *DYNC1LI2* | *Dync1li2* | DYNC1LI2 |
| *DYNLT1* | *Dynlt1* | DYNLT1 |
| *DYNLT3* | *Dynlt3* | DYNLT3 |
| *DYNLRB1* | *Dynlrb1* | DYNLRB1 |
| *DYNLRB2* | *Dynlrb2* | DYNLRB2 |
| *DYNLL1* | *Dynll1* | DYNLL1 |
| *DYNLL2* | *Dynll2* | DYNLL2 |

| (cytoplasmic dynein 2 complex) | | |
|---|---|---|
| *DYNC2H1* | *Dync2h1* | DYNC2H1 |
| *DYNC2LI1* | *Dync2li1* | DYNC2LI1 |

The term "modulator" encompasses any substance, including, without limitation, organic molecules; biomolecules (e.g., peptides, proteins, antibodies, nucleic acid oligomers, etc.); and combinations of substances, such as botanical extracts. The modulators modulate the cellular levels of dyneins, by which is meant that the cellular levels of dynein protein are either increased or decreased by the active agent. The term "modulation" may refer to up-regulation, induction, stimulation, potentiation, and/or relief of inhibition, as well as inhibition, attenuation and/or down-regulation or suppression. The modulators may be, without limitation, inhibitors or antagonists, which are, for example, compounds that bind to, partially or totally block stimulation, decrease, prevent, delay activation, inactivate, desensitize, or down-regulate expression levels of genes or dynein proteins or peptides. The modulators may also be, without limitation, activators or agonists, which are compounds that, for example, bind to, stimulate, increase, open, activate, facilitate, enhance activation, sensitize, or up-regulate expression levels of genes or dynein proteins or peptides. The mechanism by which the protein level is modulated is not important.

As used herein, the term "expression levels" refers to an amount of a gene and/or protein that is expressed in a cell. As used herein, a "gene" includes a polynucleotide containing at least one open reading frame that is capable of encoding a particular polypeptide. As used herein, the terms "polynucleotide" is synonymous with "oligonucleotide" and includes polymeric forms of nucleotides of any length, either deoxyribonucleotides or ribonucleotides, or analogs thereof, including, without limitation, mRNA, DNA, cDNA, primers, probes, and the like.

The discovery of the correlation between the expression levels of dynein and dermatological aging has led to a screening method for identifying potential dynein modulators. In one embodiment, an assay is provided for determining the expression levels of dynein after a cell has been treated, incubated, or otherwise contacted with a candidate substance. The term "candidate substance" refers to any substance that is tested for activity as a modulator of dynein, whether or not the substance is suspected of possessing such activity. The cell can be any cell that expresses dynein, preferably cytoplasmic dynein. In one embodiment, the cell is a mammalian fibroblast. In another embodiment, the cell is a mammalian keratinocyte. Preferably, the cell is a human or mouse cell. After the cell has been incubated with a candidate substance for a sufficient length of time to provide a measurable change in expression levels, which will typically be at least one hour, and more typically from about 12 hours to about 72 hours, it is then lysed to release the cellular components, such as dynein and mRNA encoding dynein. The amount of dynein protein or any subunit thereof may then be measured by any suitable technique for detection and quantitation of peptides and proteins and/or polynucleotides (e.g., mRNA).

The preferred methods for measuring expression levels of dynein involve the quantitation of mRNA expression. Suitable methods for determining mRNA expression include quantitative PCR (QPCR), real-time QPCR, reverse transcription PCR (RT-PCR), and quantitative reverse transcription PCR (QRT-PCR), as are well-known in the art. As described in detail in U.S. Patent Nos. 7,101,663 and 7,662,561, a quantitative reverse transcriptase polymerase chain reaction (QRT-PCR) for detecting mRNA may include the steps of: (a) incubating an RNA sample from the cellular lysate with a reverse transcriptase and a high concentration of a target sequence-specific reverse transcriptase primer under conditions suitable to generate cDNA; (b) subsequently adding suitable polymerase chain reaction (PCR) reagents to the reverse transcriptase reaction, including a high concentration of a PCR primer set specific to the cDNA and a thermostable DNA polymerase to the reverse transcriptase reaction; and (c) cycling the PCR reaction for a desired number of cycles and under suitable conditions to generate PCR products ("amplicons") specific to the cDNA. The products of the QRT-PCR process may be compared after a fixed number of PCR cycles to determine the relative quantity of the RNA species as compared to a given reporter gene, for example, by Southern blotting. More typically, the progress of the PCR reaction is monitored by analyzing the relative rates of amplicon production for each PCR primer set, for example, by (1) nonspecific fluorescent dyes that intercalate with any double-stranded DNA, and/or (2) sequence-specific DNA probes consisting of oligonucleotides that are labeled with a fluorescent reporter which permits detection only after hybridization of the probe with its complementary DNA target.

The mRNA may be any mRNA that is associated with dynein, including the mRNAs encoding the subunits identified in Table 1. In one embodiment, the mRNA encodes the heavy chain subunit DYNC1H1, which is synonymously known in the literature as "cytoplasmic dynein 1 heavy chain" or "conventional cytoplasmic dynein heavy chain," among others. In a preferred embodiment, the mRNA encodes human DYNC1H1.

The level of mRNA expression may be compared to controls that are not treated with the candidate substance to determine the relative degree of modulation. In some embodiments, the candidate substance will up-regulate mRNA expression by at least about 10%, more suitably at least about 20%, at least about 30%, at least about 40%, or at least about 50%. Preferably, the candidate substance will up-regulate mRNA expression by at least about 60%, at least about 70%, at least about 80%, at least about 90%, or at least about 100%. In other embodiments, the candidate substance will down-regulate mRNA expression by at least about 10%, more suitably at least about 20%, at least about 30%, at least about 40%, or at least about 50%. Candidate substances meeting these criteria may be selected for use of for further evaluation.

The dynein modulators identified by the foregoing screening protocol may be used as active agents in cosmetic preparation and may be formulated with other cosmetically acceptable components, such as a vehicle, into a composition for topical application to the skin. The compositions are topically applied to the skin in effective amounts, by which is meant an amount sufficient to achieve a measurable improvement in skin health or reduction in one or more dermatological signs of aging. Such signs of skin aging include without limitation, the following:
(a) treatment, reduction, and/or prevention of fine lines or wrinkles,
(b) reduction of skin pore size,
(c) improvement in skin thickness, plumpness, and/or tautness;
(d) improvement in skin suppleness and/or softness;
(e) improvement in skin tone, radiance, and/or clarity;
(f) improvement in procollagen and/or collagen production;
(g) improvement in maintenance and remodeling of elastin;
(h) improvement in skin texture and/or promotion of retexturization;
(i) improvement in skin barrier repair and/or function;
(j) improvement in appearance of skin contours;
(k) restoration of skin luster and/or brightness;
(l) replenishment of essential nutrients and/or constituents in the skin;
(m) decreased by aging and/or menopause;
(n) improvement in skin moisturization;
(o) increase in skin elasticity and/or resiliency;
(p) treatment, reduction, and/or prevention of skin sagging and/or
(q) reduction of pigment spots.

In practice, the compositions of the invention, including agents that modulate dynein expression levels, alone, or in cosmetically acceptable vehicles, are applied to skin in need of treatment. That is, skin which suffers from a deficiency or loss in any of the foregoing attributes or which would otherwise benefit from improvement in any of the foregoing skin attributes. The skin is typically treated once or twice daily. The treatment may continue for a week, two weeks, four weeks, eight weeks, six months or longer.

In one embodiment the active agents are topically applied, in a cosmetically acceptable vehicle, to skin suffering from fine lines and/or wrinkles to prevent, treat, and/or amelioration the appearance of the fine lines and/or wrinkles in the skin. In this case, the compositions are applied to skin in need of treatment, by which is meant skin already having wrinkles and/or fine lines or skin that is at risk of developing fine lines and/or wrinkles. Preferably, the compositions are applied directly to the fine lines and/or wrinkles on the skin of the face, neck, chest, and/or hands. Preferred agents according to this embodiment are up-regulators of dynein expression. In a preferred embodiment, the up-regulators of dynein expression lead to an enhanced production of collagen, estastin and/or fibrillin in dermal fibroblasts.

In one embodiment, the invention is directed to a method of improving the aesthetic appearance of skin by increasing the production of collagen, elastin, and/or fibrillin in the skin, the method comprising topically applying to an area of the skin in need thereof an effective amount of an agent that up-regulates dynein expression, wherein said compound has been identified for use by an assay which determines the ability of a substance to modulate expression levels of dynein, including the assay described herein. Preferably, the assay measures dynein mRNA expression levels in dermal fibroblasts.

In one embodiment, the dynein modulator comprises a natural plant material such as a botanical extract. Among the natural plant materials and botanical extracts which up-regulate dynein mention may be made of extracts from the following species: *Orthosiphon grandiflorus, Clinacanthus nutans, Cistanche tubulosa, Erythrina indica, Operculina turpethum, Gynandropsis gynandra, Erthrina Flabelliformis, Helichrysum Odoratissimum, Desmanthus illinoensis, Ozothamnus Obcordatus, Tagetes erecta Linn, Caesalpinia sappan Linn, Medemia nobilis, Calatropis gigantean, Loropetalum chinense, Heliotropium indicum, Dianella ensifolia, Breynia fruticosa, Dendranthema indicum, Sambucus chinensis, Scoparis dulcis, Acacia melanoxylon, Clerodendrum floribundum, Commersonia bartramia, Dodonaea viscose, Duboisa myoporoides, Ehretia acuminate, Ficus coronata, Goodenia ovata, Hymenosporum flavum, Lavatera plebeian, Melaleuca quinquernervia, Omolanthes populifolius*, and combinations thereof.

Other substances which up-regulate dynein include the following: 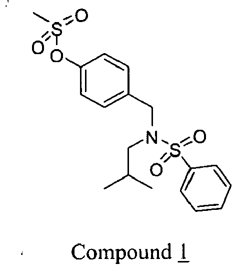 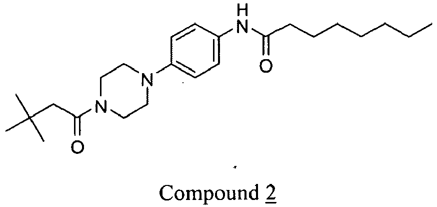 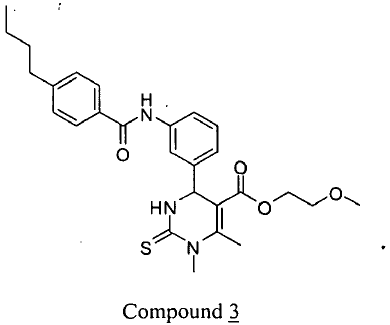 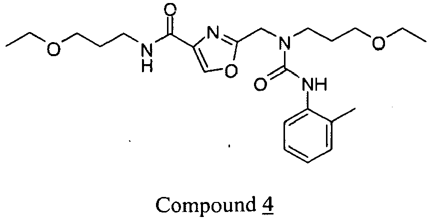 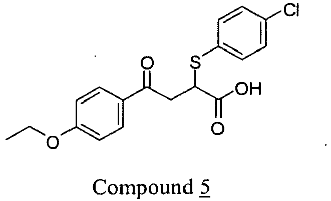 Also suitable to up-regulate dynein is chalmicrin, which is the 10-methyl-*trans-*monocyclofarnesol linked to d-mannitol as an allyl ether isolated from *Chalara microspora*; namely, 1-O-[(E)-3-Methyl-5-(2,5,6,6-tetramethyl-1-cyclohexen-1-yl)-2-pentenyl]-D-mannitol.

In one embodiment, the invention is directed to a method of reducing the appearance of age spots and other manifestations of hyperpigmentation in the skin, the method comprising topically applying to an area of the skin in need thereof an effective amount of an agent that down-regulates dynein expression, wherein said compound has been identified for use by an assay which determines the ability of a substance to modulate expression levels of dynein, including the assay described herein. Preferably, the assay measures dynein mRNA expression levels in dermal keratinocytes. Down-regulators of dynein may also be effective in the treatment of proliferative disorders or the skin, including psoriasis.

Among the natural plant materials and botanical extracts which down-regulate dynein are *Amorphophallus campanulatus, Gomphrena globosa Linn., Fibraretinum resica Pierre* (Fibrauretine), *Vernonia cinerea Linn. Less, Cayratia japonica, Pteris semipinnata,* and combinations thereof.

Other substances which down-regulate dynein include 5-chloro-salicylic acid, 3,6-Nonadienol, cis-6-nonenol and the following:

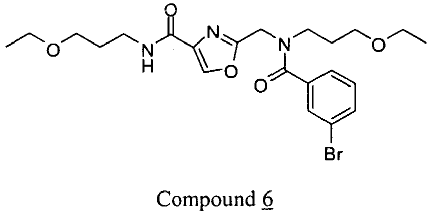

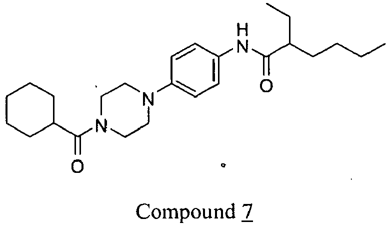

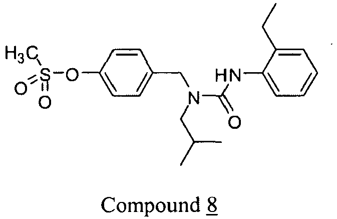

In embodiment, a method for improving the aesthetic appearance of human skin and/or improving the appearance of aged and/or photo-damaged skin comprises topically applying an effective amount of a composition comprising a natural plant extract selected from the group consisting of *Mammea siamensis* (flower extract), *Medemia nobilis* (seed extract), *Raphia farinifera* (seed extract), *Erythrina indica* (leaf extract), *Clerodendron fragrans* (whole plant extract), *Operculina turpethum* (whole plant extract, no roots), or combinations thereof, and a cosmetically acceptable vehicle.

The plant materials may be in any form including, but not limited to, the whole plant, a dried plant, a ground plant, or parts thereof, including but not limited to, seeds, needles, leaves, roots, bark, cones, stems, rhizomes, callus cells, protoplasts, flowers, and meristems, or components and/or constituents found in, or isolated from, the natural plant material, and/or portions of the plant, or any combinations thereof. In one embodiment, the natural plant material is in the form of an extract derived from the whole plant or from a select portion of the plant, such as the leaves of the plant. It is to be understood that "natural plant material" also includes an ingredient, component, constituent, or extract derived from the natural plant material. In another embodiment, the plant extract as used herein, also includes "synthetic" extracts, *i.e.*, various combinations of known plant components and/or constituents that are combined to substantially mimic the composition and/or activity of a plant extract of natural origin. Such synthetic extracts are included in the term "plant extract." The synthetic extracts will have two or more, three or more, or four or more active ingredients in common with a plant. Most preferably, the synthetic extracts will have substantially the same number of active ingredients as a natural extract. The correspondence of the numerical incidence of active ingredients between the synthetic extracts and the plant or a natural extract may also be described in terms of "percent commonality." Preferably, the synthetic extract has about 50 percent or more commonality to the chemical composition of a plant or natural extract. In other words, the synthetic extract has about 50 percent or more of the active ingredients found in the plant or a natural extract. More preferably, the chemical composition of the synthetic extract has about 70 percent or more commonality to the chemical composition of a plant or a natural extract. Optimally, a synthetic extract has about 90 percent or more commonality to the chemical composition of a plant or a natural extract.

For use in the compositions of this disclosure, the plant extract or components and/or active constituents are preferably derived directly from the plant. The components may be in a pure form, a semi-pure form, or unpurified form. In one embodiment, the components are in the form of an extract obtained by aqueous or organic solvent extraction. Non-limiting examples of organic solvents include acetic acid, ethyl acetate, lower alcohols (e.g., methanol, ethanol, isopropanol, butanol), dichloromethane, hexane, toluene, xylene, petroleum ether, and combinations thereof. The solvent may be either polar or non-polar, protic or aprotic, water-miscible or water-immiscible. The pH may be acidic, neutral, or alkaline. Well-known methods in the art may be used for aqueous or organic solvent extraction. An extraction time between about 1-8 hours at a temperature between about 30°C to about 90 °C is typically suitable. The collected extract is then fine-filtered to remove debris, and may be used directly, or is concentrated, for example by distilling the solvent or by other conventional processing, and the extract can also be provided in powder form.

The cosmetic compositions according to the invention can be formulated in a variety of forms for topical application and will comprise from about 0.00001% to about 90% by weight of one or more actives that modulate dynein, and preferably will comprise such actives in an amount from about 0.001% to about 25% by weight, and more preferably from about 0.001% to about 1% by weight.

The compositions can include a cosmetically acceptable vehicle. Such vehicles may take the form of any known in the art suitable for application to skin and may include, but are not limited to, water; vegetable oils; mineral oils; esters such as octal palmitate, isopropyl myristate and isopropyl palmitate; ethers such as dicapryl ether and dimethyl isosorbide; alcohols such as ethanol and isopropanol; fatty alcohols such as cetyl alcohol, cetearyl alcohol, stearyl alcohol and biphenyl alcohol; isoparaffins such as isooctane, isododecane and is hexadecane; silicone oils such as cyclomethicone, hydrocarbon oils such as mineral oil, petrolatum, isoeicosane and polyisobutene; polyols such as propylene glycol, glycerin, butylene glycol, pentylene glycol and hexylene glycol; liposomes; waxes; or any combinations or mixtures of the foregoing.

The vehicle may comprise an aqueous phase, an oil phase, an alcohol, a silicone phase or mixtures thereof and may be in the form of an emulsion. Non-limiting examples of suitable emulsions include water-in-oil emulsions, oil-in-water emulsions, silicone-in-water emulsions, water-in-silicone emulsions, glycerin-in-oil emulsions, wax-in-water emulsions, water-oil-water triple emulsions or the like. The emulsion may include an emulsifier, such as a nonionic, anionic or amphoteric surfactant, or a gelling agent.

The topical composition will typically have a pH range from about 2 to about 8, with a pH in the range of from 3 to 7 being preferred. In some embodiment, the composition will have a pH in the range of from 3.5 to 5.5. Suitable pH adjusters such as citric acid and triethanolamine may be added to bring the pH within the desired range.

In one embodiment of the invention, the compositions may include additional skin actives, including but not limited to, botanicals, keratolytic agents, desquamating agents, keratinocyte proliferation enhancers, collagenase inhibitors, elastase inhibitors, depigmenting agents, anti-inflammatory agents, steroids, anti-acne agents, antioxidants, and advanced glycation end-product (AGE) inhibitors.

The composition may comprise additional active ingredients having anti-aging benefits, as it is contemplated that synergistic improvements may be obtained with such combinations. Exemplary anti-aging components include, without limitation, botanicals (e.g., Butea Frondosa extract); phytol; thiodipropionic acid (TDPA) and esters thereof; retinoids (e.g., 9-cis retinoic acid, 13-cis retinoic acid, all-trans retinoic acid and derivatives thereof, phytanic acid, retinol (Vitamin A) and esters thereof, such as retinol palmitate, retinol acetate and retinol propionate, and salts thereof and others); hydroxy acids (including alpha-hydroxyacids and beta-hydroxyacids), salicylic acid and alkyl salicylates; exfoliating agents (e.g., glycolic acid, 3,6,9-trioxaundecanedioic acid, etc.), estrogen synthetase stimulating compounds (e.g., caffeine and derivatives); compounds capable of inhibiting 5 alpha-reductase activity (e.g., linolenic acid, linoleic acid, finasteride, and mixtures thereof); and barrier function enhancing agents (e.g., ceramides, glycerides, cholesterol and its esters, alpha-hydroxy and omega-hydroxy fatty acids and esters thereof, etc.), to name a few. Exemplary retinoids include, without limitation, retinoic acid (*e.g*., all-trans or 13-cis) and derivatives thereof, retinol (Vitamin A) and esters thereof, such as retinol palmitate, retinol acetate and retinol propionate, and salts thereof.

In another embodiment, the topical compositions of the present invention may also include one or more of the following: a skin penetration enhancer, an emollient, such as isopropyl myristate, petrolatum, silicones (*e.g*., methicone, dimethicone), oils, mineral oils, and fatty acid esters; a humectant, such as glycerin or caprylyl glycol, a skin plumper, such as palmitoyl oligopeptide, collagen, or collagen and/or glycosaminoglycan (GAG) enhancing agents, a sunscreen, such as avobenzone, an exfoliating agent, and an antioxidant.

Suitable exfoliating agents include, for example, alpha-hydroxyacids, beta-hydroxyacids, oxa-acids, oxadiacids, and their derivatives such as esters, anhydrides and salts thereof. Suitable hydroxy acids include, for example, glycolic acid, lactic acid, malic acid, tartaric acid, citric acid, 2-hydroxyalkanoic acid, mandelic acid, salicylic acid and derivatives thereof. A preferred exfoliating agent is glycolic acid. When present, the exfoliating agent may comprise from about 0.1 wt % to about 80 wt % of the composition.

Examples of antioxidants that may be used in the present compositions include compounds having phenolic hydroxy functions, such as ascorbic acid and its derivatives/esters; beta-carotene; catechins; curcumin; ferulic acid derivatives (*e.g*. ethyl ferulate, sodium ferulate); gallic acid derivatives (*e.g.*, propyl gallate); lycopene; reductic acid; rosmarinic acid; tannic acid; tetrahydrocurcumin; tocopherol and its derivatives; uric acid; or any mixtures thereof. Other suitable antioxidants are those that have one or more thiol functions (-SH), in either reduced or non-reduced form, such as glutathione, lipoic acid, thioglycolic acid, and other sulfhydryl compounds. The antioxidant may be inorganic, such as bisulfites, metabisulfites, sulfites, or other inorganic salts and acids containing sulfur. Compositions of the present invention may comprise an antioxidant preferably from about 0.001 wt % to about 10 wt%, and more preferably from about 0.01 wt% to about 5 wt%, of the total weight of the composition.

Other conventional additives include: vitamins, such as tocopherol and ascorbic acid; vitamin derivatives such as ascorbyl monopalmitate; thickeners such as hydroxyalkyl cellulose; gelling agents; structuring agents, metal chelating agents such as EDTA; pigments; colorants, and pH adjusters. The composition may optionally comprise other components known to those skilled in the art including, but not limited to, film formers, moisturizers, minerals, viscosity and/or rheology modifiers, anti-acne agents, insect repellents, skin cooling compounds, skin protectants, lubricants, fragrances, preservatives, stabilizers, and mixtures thereof. In addition to the foregoing, the cosmetic compositions of the invention may contain any other compound for the treatment of skin disorders.

The composition may be formulated in a variety of product forms, such as, for example, an emulsion, lotion, cream, serum, spray, aerosol, cake, ointment, essence, gel, paste, patch, pencil, towelette, mask, stick, foam, elixir, concentrate, and the like, particularly for topical administration. Preferably the composition is formulated as an emulsion, lotion, cream, ointment, serum or gel.

The invention provides a method for treating aging skin by topically applying a composition comprising an active agent that modulates dynein, preferably in a cosmetically acceptable vehicle, over the affected area for a period of time sufficient to reduce, ameliorate, reverse or prevent dermatological signs of aging.

Generally, the improvement in the condition and/or aesthetic appearance is selected from the group consisting of: reducing dermatological signs of chronological aging, photo-aging, hormonal aging, and/or actinic aging; preventing and/or reducing the appearance of lines and/or wrinkles; reducing the noticeability of facial lines and wrinkles, facial wrinkles on the cheeks, forehead, perpendicular wrinkles between the eyes, horizontal wrinkles above the eyes, and around the mouth, marionette lines, and particularly deep wrinkles or creases; improving the appearance of suborbital lines and/or periorbital lines; reducing the appearance of crow's feet; rejuvenating and/or revitalizing skin, particularly aging skin; reducing skin fragility; preventing and/or reversing of loss of glycosaminoglycans and/or collagen; ameliorating the effects of estrogen imbalance; preventing skin atrophy; preventing, reducing, and/or treating hyperpigmentation or hypopigmentation; minimizing skin discoloration; improving skin tone, radiance, clarity and/or tautness; preventing, reducing, and/or ameliorating skin sagging; improving skin firmness, plumpness, suppleness and/or softness; improving procollagen and/or collagen production; improving skin texture and/or promoting retexturization; improving skin barrier repair and/or function; improving the appearance of skin contours; restoring skin luster and/or brightness; minimizing dermatological signs of fatigue and/or stress; resisting environmental stress; replenishing ingredients in the skin decreased by aging and/or menopause; improving communication among skin cells; increasing cell proliferation and/or multiplication; increasing skin cell metabolism decreased by aging and/or menopause; retarding cellular aging; improving skin moisturization; enhancing skin thickness; slowing or halting skin thinning; increasing skin elasticity and/or resiliency; enhancing exfoliation; improving microcirculation; decreasing and/or preventing cellulite formation; and any combinations thereof.

In one embodiment, the compositions will comprise from about 0.00001% to about 20%, more typically from about 0.001% to about 10%, including from about 0.01 to about 1% by weight of a modulator on dynein. Preferably, the modulator may be an up-regulator of dynein in fibroblasts. Also preferred, the modulator may be an up-regulator of dynein in keratinocytes. The modulator may be a down-regulator of dynein in keratinocytes. Less preferred, although still within the scope of the invention, the modulator may be a down-regulator of dynein in fibroblasts. In one embodiment, the modulator is an up-regulator of dynein in both fibroblasts and keratinoctyes. Combinations of modulators are also contemplated. In one embodiment, the modulator is a combination of two or more substances that are up-regulators of dynein in fibroblasts and/or keratinoctyes.

The composition will typically be applied to the skin one, two, or three times daily for as long as is necessary to achieve desired results. The treatment regiment may comprise daily application for at least one week, at least two weeks, at least four weeks, at least eight weeks, or at least twelve weeks or more. Chronic treatment regimens are also contemplated. The effect of a composition on the formation or appearance of fine lines and wrinkles can be evaluated qualitatively, *e.g.*, by visual inspection, or quantitatively, *e.g.*, by microscopic or computer assisted measurements of wrinkle morphology (e.g., the number, depth, length, area, volume and/or width of wrinkles per unit area of skin). In one embodiment, the composition of the invention will be applied to the skin in an amount from about 0.001 to about 100 mg/cm², more typically from about 0.01 to about 20 mg/cm², and preferably about 0.1 to about 10 mg/cm².

It is also contemplated that the compositions of the invention will be useful for treating thin skin by topically applying the composition to thin skin of an individual in need thereof. "Thin skin" is intended to include skin that is thinned due to chronological aging, menopause, or photo-damage and skin that is thinning prematurely. In some embodiments, the treatment is for thin skin in men, whereas other embodiments treat thin skin in women, pre-menopausal or post-menopausal, as it is believed that skin thins differently with age in men and women, and in particular in women at different stages of life.

The method of the invention may be employed prophylactically to forestall aging including in individuals that have not manifested signs of skin aging, most commonly in individuals under 25 years of age. The method may also reverse or treat signs of aging once manifested as is common in individuals over 25 years of age, or to slow the progression of dermatological aging in such individuals.

### EXAMPLES

The following examples describe specific aspects of the invention to illustrate the invention but should not be construed as limiting the invention, as the examples merely provide specific methodology useful in the understanding and practice of the invention and its various aspects.

### Example 1

### Age-related expression of dynein in dermal fibroblasts.

Normal dermal fibroblasts from neonatal (n=3) and female donors aged 31-39 (n=3) were plated on 10 cm dishes in DMEM (10% FBS, 4.5g/L glucose) and grown to subconfluence. Cells were washed with cold PBS and RNA was isolated using RNAqueous kit according to the manufacturer's instructions. RNA integrity and concentration were verified by Agilent Bioanalyzer 2100 using RNA Nano Chip. Gene expression analysis was performed using the isolated RNA and an ABI 7900 HT RT-PCR machine. The dynein probe used was *Homo sapiens,* cytoplasmic, heavy chain, DYNC1H1, with ABI code DYNC1H1-Hs00300243_m1. Internal control was included and dynein gene expression was normalized against GAPDH. A significant (∼14%) reduction of dynein expression was found in cells from 30+ yr-old donors compare to neonatal cells. The intrinsic (normalized) gene expression levels are provided below in Table 1.

In another experiment, human biopsies were analyzed for expression of DYNC1H1 after exposure to UV irradiation. Exploratory biopsy skin biopsies from dorsal forearms (photo-exposed) and upper under arms (photo-protected) of young (aged 18-25 year-old, n=15) and old (aged 45-65 year-old, n=15) healthy female caucasian donors were collected, formalin-fixed and paraffin embedded. Five micron sections were cut and placed on slides. IHC was performed using rabbit polyclonal antibody against dynein and was visualized using vector blue conjugated goat anti-rabbit secondary antibody. From the younger cohort, 62% of the subjects showed increased expression of dynein, whereas only 33% of older subjects showed an increase. These date demonstrate that older skin is much less effective at producing dynein when it is needed. The increase in gene expression levels on UV exposure are also provided below in Table 2.

**Table 2.**

| Age | Normalized expression of DYNC1H1 | % of individuals showing DYNC1H1 increase with UV exposure |
|---|---|---|
| Young | 1.61 | 62 |
| Old | 1.39 | 33 |

The data in Table 2 indicate that cytoplasmic dynein expression decreases with age, as does the skin's ability to up-regulate dynein expression in response to as stressor such as UV exposure. It is therefore believed that modulation of dynein is a rational approach to combating age and environmental-related aging of the skin.

### EXAMPLE 2

### Dynein modulation assay.

Normal human dermal fibroblasts or keratinocytes were cultured in 96-well tissue culture treated plates, containing appropriate culture medium. Stock solution of actives were made in an appropriate vehicle (water/Ethanol/Propylene glycol). Cells were treated with test material or respective vehicle control diluted in growth medium for 24 hours in a humidified 37°C incubator with 10% CO₂. After incubation, growth medium from each plate was removed and 100 µL of lysis buffer was added to the wells and placed in 37°C incubator with 10% CO₂ for 30 minutes. At the end of incubation, the cells are collected in freezer plates and placed in -80°C freezer, until analysis. Changes in mRNA for Dynein after treatment were analyzed using Panomics Quantigene multiplex assay that employs a branched DNA technology, following manufacturer's instructions (Affymetrix, CA). Percent increase (up-regulation) or decrease (down-regulation) in mRNA for the heavy chain DYNC1H1 of cytoplasmic dynein was calculated by comparing the test results to the control. The percent up-regulation or down-regulation is converted to a scaled score as shown below in Table 3.

**Table 3.**

| % Up-regulation | Up-regulation Scale | % Down-regulation | Down-regulation Scale |
|---|---|---|---|
| 0-20 | 0 | 0-20 | 0 |
| 21-40 | + | 21-40 | - |
| 41-60 | ++ | 41-60 | -- |
| 61-80 | +++ | 61-80 | --- |
| >81 | ++++ | >81 | ---- |

### EXAMPLE 3

### Up-regulation of dyneins.

A variety of botanical extract were tested for the ability to up-regulate dynein according to the method of Example 2. The results are provided below in Table 4. The concentrations of each extract are provided based on the dry weight of the given plant extract, by which is meant the weight of the extract after volatile extraction solvents have been removed. The cells tested were either keratinocytes (K) or fibroblasts (F).

**Table 4.**

| Plant Extract | Conc. (%) | Cell Type | Effect |
|---|---|---|---|
| *Orthosiphon grandiflorus* | 0.0001 | K | +++ |
| *Clinacanthus nutans* | 0.01 | K | ++++ |
| *Cistanche tubulosa* | 0.01 | K | ++++ |
| *Erythrina indica* | 0.1 | K | +++ |
| *Erythrina indica* | 0.01 | F | + |
| *Erythrina indica* | 0.001 | F | + |
| *Operculina turpethum* | 0.1 | K | ++++ |
| *Gynandropsis gynandra* | 0.1 | K | +++ |
| *Erthrina Flabelliformis* | 0.01 | F | ++++ |
| *Erthrina Flabelliformis* | 0.001 | K | ++++ |
| *Helichrysum Odoratissimum* | 0.01 | K | ++++ |
| *Desmanthus illinoensis* | 0.01 | F | ++++ |
| *Ozothamnus Obcordatus* | 0.01 | K | +++ |
| *Tagetes erecta Linn* | 0.01 | K | ++ |
| *Tagetes erecta Linn* | 0.001 | K | +++ |
| *Caesalpinia sappan Linn* | 0.01 | K | ++++ |
| *Medemia nobilis* | 0.01 | K | ++++ |
| *Calatropis gigantea* | 0.001 | F | ++++ |
| *Calatropis gigantea* | 0.0001 | F | ++++ |
| *Loropetalum chinense* | 0.1 | K | +++ |
| *Heliotropium indicum* | 0.1 | K | +++ |
| *Heliotropium indicum* | 0.01 | K | + |
| *Dianella ensifolia* | 0.1 | K | +++ |
| *Breynia fruticosa* | 0.1 | K | +++ |
| *Dendranthema indicum* | 0.1 | K | +++ |
| *Dendranthema indicum* | 0.01 | K | + |
| *Sambucus chinensis* | 0.1 | K | ++++ |
| *Sambucus chinensis* | 0.01 | K | + |
| *Scoparis dulcis* | 0.1 | K | ++++ |
| *Scoparis dulcis* | 0.01 | K | ++ |
| *Acacia melanoxylon* | 0.001 | K | +++ |
| *Clerodendrum floribundum* | 0.01 | K | ++++ |
| *Clerodendrum floribundum* | 0.001 | K | + |
| *Commersonia bartramia* | 0.001 | K | +++ |
| *Dodonaea viscosa* | 0.01 | F | ++++ |
| *Dodonaea viscosa* | 0.001 | K | + |
| *Dodonaea viscosa* | 0.0001 | K | +++ |
| *Duboisa myoporoides* | 0.01 | F | ++++ |
| *Duboisa myoporoides* | 0.01 | K | ++++ |
| *Duboisa myoporoides* | 0.001 | F | ++ |
| *Duboisa myoporoides* | 0.001 | K | ++++ |
| *Ehretia acuminate* | 0.01 | K | ++ |
| *Ehretia acuminate* | 0.001 | F | ++++ |
| *Ehretia acuminate* | 0.001 | K | + |
| *Ficus coronata* | 0.001 | F | + |
| *Ficus coronata* | 0.001 | K | ++ |
| *Ficus coronata* | 0.0001 | F | +++ |
| *Ficus coronata* | 0.0001 | K | + |
| *Goodenia ovata* | 0.01 | F | ++ |
| *Goodenia ovata* | 0.001 | K | ++++ |
| *Goodenia ovata* | 0.0001 | K | +++ |
| *Hymenosporum flavum* | 0.01 | F | + |
| *Hymenosporum flavum* | 0.001 | F | ++++ |
| *Hymenosporum flavum* | 0.001 | K | ++++ |
| *Lavatera plebeia* | 0.01 | F | + |
| *Lavatera plebeia* | 0.01 | K | ++++ |
| *Melaleuca quinquernervia* | 0.001 | K | ++++ |
| *Melaleuca quinquernervia* | 0.0001 | K | +++ |
| *Omolanthes populifolius* | 0.0001 | K | +++ |
| *Melicope hayesii* | 0.01 | K | ++++ |
| *Medemia nobilis* | 0.01 | K | ++++ |

Compounds 1-5 and chalmicrin were also tested for the ability to up-regulate dynein according to the method of Example 2. The results are reported in Table 5.

**Table 5.**

| Plant Extract | Conc. (%) | Cell Type | Effect |
|---|---|---|---|
| Compound 1 | 0.00005 | F | ++ |
| Compound 2 | 0.0005 | K | ++ |
| Compound 3 | 0.00005 | F | ++ |
| Compound 4 | 0.00005 | F | ++ |
| Compound 5 | 0.00001 | F | ++ |
| Chalmicrin | 0.0001 | K | ++++ |

### EXAMPLE 4

### Down-regulation of dyneins.

A variety of compounds and botanical extracts were tested for the ability to down-regulate dynein according to the method of Example 2. The results are provided below in Table 6, where the concentrations of the botanical extracts are provided based on the dry weight of the given plant extract. In each case statistical significance was achieved at the p < 0.05 level or better.

**Table 6.**

| Plant Extract | Conc. (%) | Cell Type | Effect |
|---|---|---|---|
| 5-Chlorosalicylic acid | 0.1 | K | -- |
| *Amorphophallus campanulatus* | 0.01 | K | - |
| *Gomphrena globosa* Linn. | 0.01 | F | - |
| Compound 6 | 0.0005 | K | - |
| Compound 7 | 0.00005 | K | - |
| Compound 8 | 0.0005 | F | - |
| 3,6-Nonadienol | 0.01 | F | - |
| *Fibraretinum resica Pierre* | 0.001 | F | - |
| *Tiliacora Triandra* | 0.0001 | F | - |
| *Vernonia cinerea* (Linn.) Less. | 0.01 | K | - |
| cis-6-Nonenol | 0.0001 | K | - |
| *Derris Scandens* | 0.001 | K | - |
| *Cayratia japonica* | 0.01 | F | - |

The salicylic acid derivative provided the greatest degree of down-regulation of mRNA for the heavy chain DYNC1H1 of cytoplasmic dynein, yielding a 45.03% reduction (p = 0.02) in keratinocytes, followed by *Tliliacora Triandra* which gave a 36.7% reduction in fibroblasts (p = 0.01).

### EXAMPLE 5

### In vivo up-regulation of dynein.

Botanical extracts were tested for the ability to up-regulate dynein in vivo. 33 healthy female Caucasian subjects aged 30-65 with skin type II or III were treated with ingredients on the dorsal forearm for 3 weeks (3 consecutive rounds of 5 x 24 hour patches under semi-occlusion). Test articles and vehicles were applied in a randomized allocation on five sites on each forearm. The application dose was 2 mg/cm². After treatment, a 2 mm punch biopsy was obtained from each treatment site and fixed in 4% paraformaldehyde and processed for IHC studies. IHC was performed using rabbit polyclonal antibody against dynein and was visualized using vector blue conjugated goat anti-rabbit secondary antibody, as described in Example 1. The results shown below in Table 7 are expressed as the number (N) of patients showing up-regulation, no change, or down-regulation in dynein levels.

**Table 7.**

| Plant Extract | Up-regulated (N) | No change (N) | Down-regulated (N) |
|---|---|---|---|
| *Melicope hayesii* | 9 | 7 | 3 |
| *Mammea siamensis* | 10 | 7 | 2 |
| *Operculina turpethum* | 3 | 13 | 4 |
| *Erythrina indica* | 6 | 7 | 4 |
| *Raphia farinifera U* | 7 | 7 | 4 |
| *Medemia nobilis X* | 5 | 11 | 3 |
| Compound 1 | 10 | 6 | 2 |

As shown in Table 7, nearly all of the botanical extracts tested up-regulate dynein in vivo when topically applied to skin. While *Operculina turpethum* did not show significant up-regulation or down-regulation in this study, based on its in vitro potency, it is believed that the results would be improved by the use of a penetration enhancer to improve delivery into the skin and/or an increased dose or treatment duration.

## Claims

1. A cosmetic method for ameliorating hyperpigmentation of human skin comprising topically applying to an area of the skin in need thereof an effective amount of an active agent selected from the group of botanical extracts of Amorphophallus campanulatus, Gomphrena globose Linn., Fibraretinum resica Pierre (Fibrauretine), Vernonia cinerea Linn. Less, Cayratia japonica, Pteris semipinnata and 5-chloro-salicylic acid, 3,6-Nonadienol, cis-6-nonenol and the compounds: 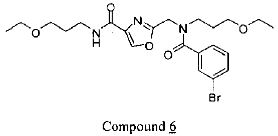 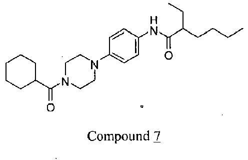 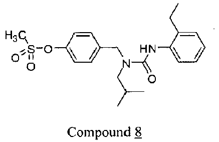 and combinations thereof.

2. A method for identifying active agents useful for ameliorating hyperpigmentation of human skin comprising assaying candidate substances for ability to down-regulate cellular levels of cytoplasmic dynein, wherein the ability of said active agent to down-regulate cytoplasmic dynein is determined by an in vitro assay which measures the expression level of cytoplasmic dynein in a human or mouse dermal keratinocyte cell that has been contacted with said active agent, wherein the in vitro assay measures the expression levels of the human gene *DYNC1H1* or the mouse gene *Dync1h1* encoding a heavy chain subunit DYNC1H1 by measuring the expression levels of the corresponding mRNA with quantitative polymerase chain reaction (qPCR); and wherein the active agent down-regulates the expression levels of the corresponding mRNA by at least 10%.

## Patentansprüche

1. Kosmetisches Verfahren zur Verbesserung der Hyperpigmentierung von menschlicher Haut, umfassend das auf einen Bereich der Haut, der dies benötigt, erfolgende topische Auftragen einer wirksamen Menge eines Wirkstoffs, der aus der Gruppe aus Pflanzenextrakten aus Amorphophallus campanulatus, Gomphrena globose Linn., Fibraretinum resica Pierre (Fibrauretin), Vernonia cinerea Linn. Less, Cayratia japonica, Pteris semipinnata und 5-Chlor-salicysäure, 3,6-Nonadienol, cis-6-Nonenol und den Verbindungen: 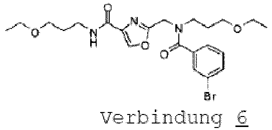 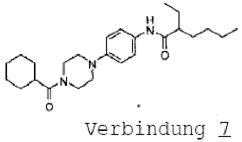 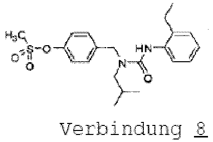 und Kombinationen davon ausgewählt ist.

2. Verfahren zur Identifikation von Wirkstoffen, die zur Verbesserung der Hyperpigmentierung von menschlicher Haut zweckdienlich sind, umfassend das Testen von Kandidatensubstanzen auf ihre Fähigkeit, die zellulären Niveaus von zytoplasmatischem Dynein hinunterzuregulieren, wobei die Fähigkeit des Wirkstoffs, das zytoplasmatische Dynein hinunterzuregulieren, durch einen in vitro-Test bestimmt wird, der das Expressionsniveau von zytoplasmatischem Dynein in einer dermalen Keratinozytenzelle des Menschen oder der Maus misst, die mit dem Wirkstoff in Kontakt gebracht wurde, wobei der in vitro-Test die Expressionsniveaus des menschlichen Gens *DYNC1H1* oder des Mausgens *Dync1h1* misst, das eine Schwerketten-Untereinheit DYNC1 H1 codiert, indem die Expressionsniveaus der entsprechenden mRNA mittels quantitativer Polymerase-Kettenreaktion (qPCR) gemessen werden; und wobei der aktive Wirkstoff die Expressionsniveaus der entsprechenden mRNA um wenigstens 10% hinunterreguliert.

## Revendications

1. Méthode cosmétique pour améliorer l'hyperpigmentation de la peau humaine comprenant l'application locale, sur une zone de la peau qui en a besoin, d'une quantité efficace d'un agent actif choisi parmi le groupe comprenant des extraits botaniques d'*Amorphophallus campanulatus, Gomphrena globosa* Linn., *Fibraretinum resica* Pierre (fibraurétine), *Vernonia cinerea* (Linn.) Less., *Cayratia japonica, Pteris semipinnata* et l'acide 5-chloro-salicylique, le 3,6-nonadiénol, le cis-6-nonénol et les composés : 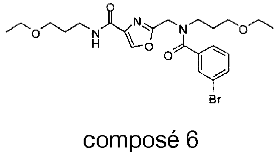 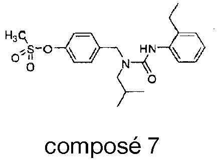 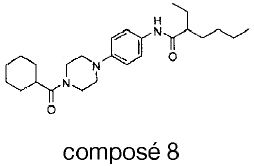 et des combinaisons de ceux-ci.

2. Méthode pour identifier des agents actifs utiles pour améliorer l'hyperpigmentation de la peau humaine, comprenant l'évaluation des substances en fonction de leur capacité de réguler à la baisse les taux cellulaires de dynéine cytoplasmique, la capacité dudit agent actif à réguler à la baisse les taux cellulaires de dynéine cytoplasmique étant déterminée par un essai *in vitro* qui mesure le niveau d'expression de la dynéine cytoplasmique dans un kératinocyte dermique humain ou de souris qui a été mis en contact avec ledit agent actif, l'essai *in vitro* mesurant les taux d'expression du gène humain *DYNC1H1* ou du gène de souris *Dync1h1* codant pour une sous-unité à chaîne lourde DYNC1 H1 par la mesure du niveau d'expression de l'ARNm correspondant par réaction en chaîne à la polymérase quantitative (RCPq), et dans laquelle l'agent actif régule à la baisse les niveaux d'expression de l'ARNm correspondant d'au moins 10 %.
